# EUROPEAN PATENT SPECIFICATION

(11) **EP 3 000 818 B1**
(45) Date of publication and mention of the grant of the patent: **27.09.2017**
(21) Application number: 14801188.5
(22) Date of filing: 23.05.2014
(51) Int. Cl.: C07H 1/00, C07H 17/07

(54) **PREPARATION METHOD OF TRIHYDROXYETHYL RUTOSIDE**
VERFAHREN ZUR HERSTELLUNG VON TRIHYDROXYETHYLRUTOSID
PROCEDE DE PREPARATION DE TRIHYDROXYETHYL RUTOSIDE

(43) Date of publication of application: 30.03.2016
(73) Proprietor: Jinan Xinlite Technology Co. Ltd, Jinan, Shandong 250101 (CN)
(72) Inventor: LI, Si, Shandong 250101 (CN); LIANG, Xutong, Shandong 250101 (CN); LI, Dapeng, Shandong 250101 (CN)
(74) Representative: Lermer, Christoph
(86) International application number: PCT/CN2014/078322
(87) International publication number: WO 2014/187364

(56) References cited:
- CN-A- 1 331 697
- CN-A- 1 554 353
- CN-A- 1 814 613
- CN-A- 101 891 784
- CN-A- 102 924 546
- GB-A- 1 497 157
- US-A- 3 420 815
- US-A- 4 153 788
- US-B1- 6 855 697

## Description

### FIELD OF THE INVENTION

This invention relates to a preparation method for a pharmaceutical compound, specifically, a preparation method of trihydroxyethyl rutoside.

### BACKGROUND OF THE INVENTION

Troxerutin, also called Venoruton, is mainly composed of 7,3',4'-trihydroxyethyl rutoside (abbreviated as "trihydroxyethyl rutoside" thereafter). The formula is C₃₃H₄₂O₁₉, CAS No. is 7085-55-4, molecular weight is 742.69, and the chemical structure is as below:

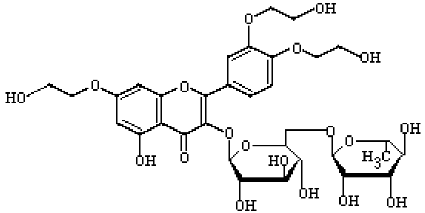

Troxerutin is a kind of anticoagulant and thrombolytic drug. Generally, it can be synthesized by reacting rutin with ethylene oxide in methanol and water as medium in the presence of a basic catalyst. Currently, active pharmaceutical ingredient of troxerutin is available on the market, sold at different purities such as 60%, 80% and 88%.

Trihydroxyethyl rutoside is the active ingredient in troxerutin. The higher its content is in troxerutin, the higher the quality of the drug. However, the current technical approach for preparing troxerutin with high purity is far from ideal.

The products of troxerutin on the market include injection formulation, oral solution and capsule. Especially for injection, the non-ideal purity of troxerutin produced by current technologies may pose great risk to clinical applications. Actually, injecting such a drug may lead to high prevalence of adverse effects, particularly allergic reactions. Improving the purity of troxerutin, specifically, the purity of the active ingredient trihydroxyethyl rutoside, therefore, has been studied by a great number of researchers.

The exiting preparation method of troxerutin utilizes a one-step reaction, i.e., direct hydroxyethylation of rutin to complete the reaction. Different hydroxyethylation techniques are based on utilizing different hydroxyethylation agents, reaction media, catalysts, reaction parameters, etc. The following are examples.
1. In Patent BG2888B1, trihydroxyethyl rutoside with purity of 85.8% was prepared using water as solvent, ammonium water as catalyst, and ethylene oxide as hydroxyethylation agent.
2. In Patent CN1331697 (US6855697), trihydroxyethyl rutoside with purity of 92% was prepared by using water as solvent, alkaline metal as catalyst, and ethylene oxide as hydroxyethylation agent, and by controlling the recrystallization condition.
3. In Patent US3420815, troxerutin with chromatographic purity of 87.4%, content of 85.3% and melting point of 181-183°C was prepared by using sodium hydroxide as catalyst.
4. In Patent CN1554353, trihydroxyethyl rutoside with content of 90% was prepared by using organic solvents (methanol and ethanol) as solvent and pyridine as catalyst.
5. In Patent CN1814613, trihydroxyethyl rutoside with content of 85% was prepared by using resin to control the pH value of the reaction solution.
6. In Patent GB 1 497 157 A, a process for preparing 7-mono-O (β-hydroxyethyl)-rutoside is disclosed.

One of the difficulties in preparing trihydroxyethyl rutoside is that rutin has four active hydroxyl groups, all of which can be hydroxyethylated, resulting in a mixture of products. Another difficulty is that natural rutin is mixed with impurities having highly similar structures, making purification difficult. It is challenging to obtain rutin with high purity by using conventional methods. Patent CN200810007927.2 discloses a method for purifying rutin by using complex reverse chromatography, which only gives gramscale rutin with purity of 98%. In fact, commercially available rutin with low purity is always used as raw materials to prepare troxerutin. The impurities in the raw materials also undergo hydroxyethylation reaction, generating impurities with highly similar properties as trihydroxyethyl rutoside, and such impurities are hard to be removed. Actually, the preparation of trihydroxyethyl rutoside with high purity is highly challenging. Except for the chromatographic method of Jun Li et al. [2004, "Separation of troxerutin as reference substance by preparative liquid chromatography", Chinese Journal of Pharmaceuticals, 35(5), 285-287] which is capable of preparing trihydroxyethyl rutoside with a purity of 99% as reference substance, no other reports of preparing trihydroxyethyl rutoside with a purity higher than 98% by using conventional methods can be found. Even though a product with a purity of 99% is claimed on the market, the preparation method is not disclosed, and the product is not available. The purity of reference substance prepared by current methods, even after multiple recrystallizations, is only 96-97%.

In order to solve the problem, this invention provides a preparation method of troxerutin with low content of impurities. Specifically, this method provides troxerutin containing less than 2% of non-troxerutin derivatives and greater than 98%of trihydroxyethyl rutoside. The essence of this invention relies on a two-step reaction to synthesize trihydroxyethyl rutoside with purification of the intermediate, whereby obtaining high-purity product.

### SUMMARY OF THE INVENTION

This invention discloses a new preparation method of troxerutin, including
(1) Preparation of 7-monohydroxyethyl rutoside from rutin;
(2) Purification of 7-monohydroxyethyl rutoside;
(3) Preparation of trihydroxyethyl rutoside by using purified 7-monohydroxyethyl rutoside; and
(4) Purification of trihydroxyethyl rutoside.

It was discovered that 7-monohydroxyethyl rutoside, as an intermediate for preparing troxerutin, is significantly different in properties in comparison to other impurities contained in the raw materials. Therefore, 7-monohydroxyethyl rutoside can be prepared with a purity of greater than 98%. Comparing with current technologies, this invention can use conventional purification methods, especially recrystallization method. No special expensive equipments or expensive separation chromatographic column is needed. This method thus greatly saves cost for manufacture and makes it possible for industrial production of trihydroxyethyl rutoside. Using conventional method is impossible to obtain trihydroxyethyl rutoside with high purity due to the similarities in properties between the impurities and the final product, even if recrystallization of the final product is performed repeatedly.

The synthetic approach in this invention can be illustrated as below:

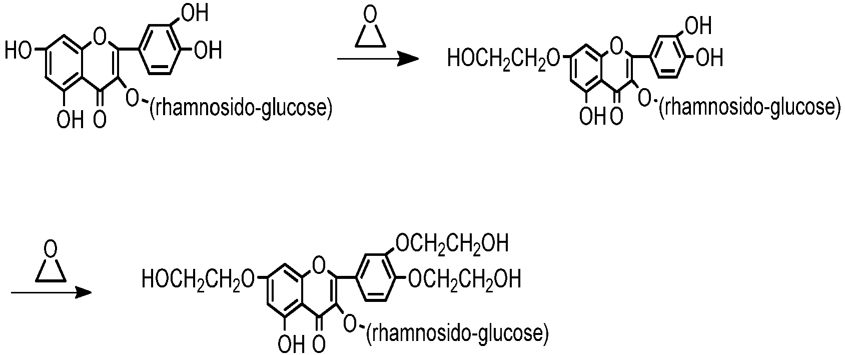

### BRIEF DESCRIPTION OF THE DRAWINGS

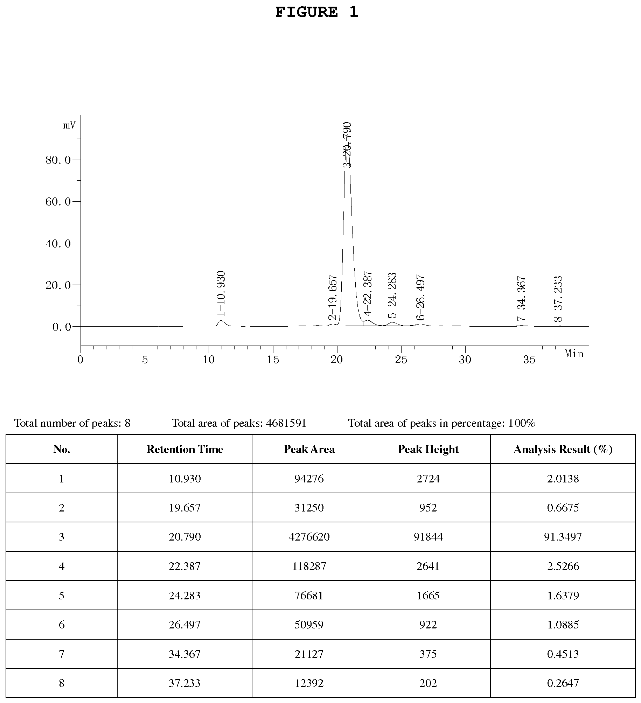

Figure 1: HPLC spectrum of 7-monohydroxyethyl rutoside reaction solution in this invention.

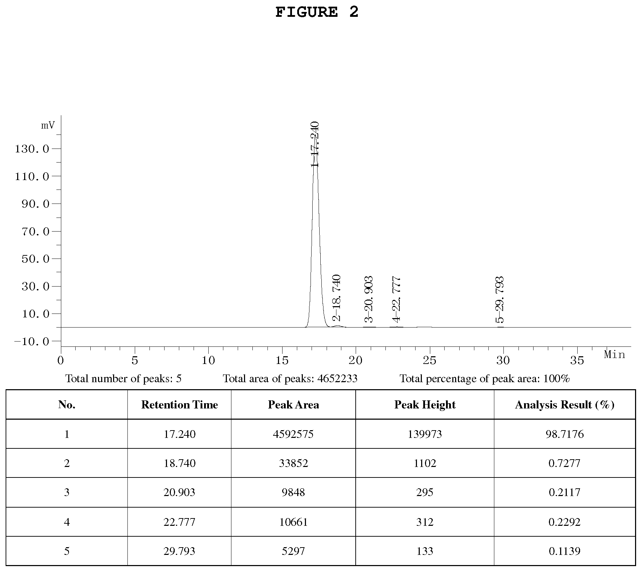

Figure 2: HPLC spectrum of 7-monohydroxyethyl rutoside after purification in this invention.

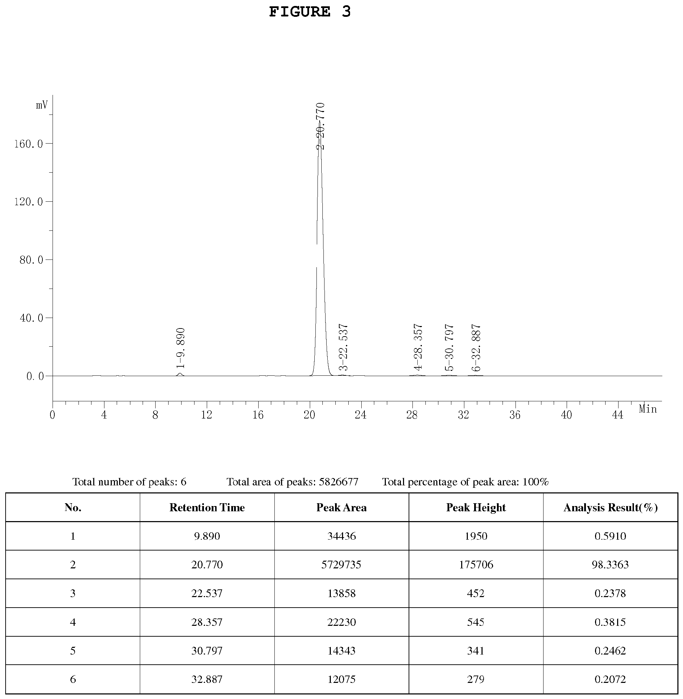

Figure 3: HPLC spectrum of trihydroxyethyl rutoside (98%) in this invention.

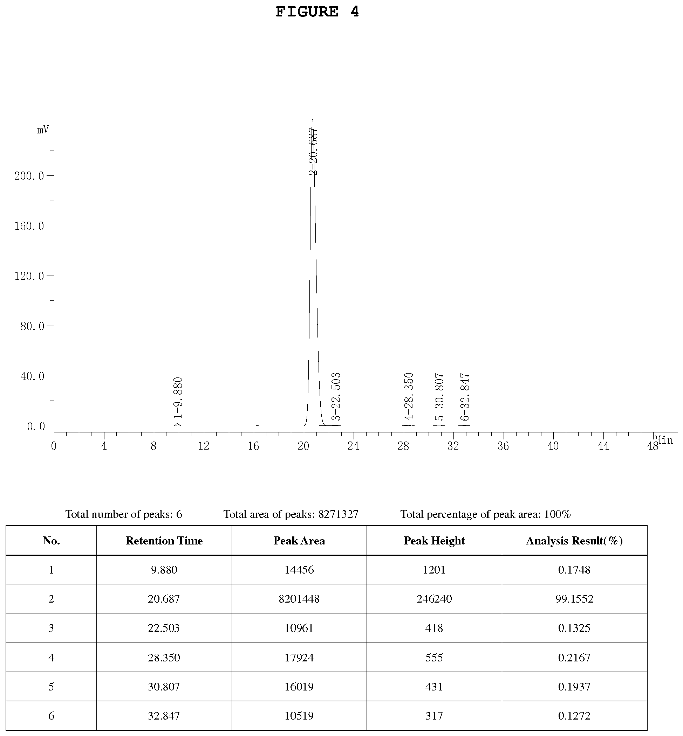

Figure 4: HPLC spectrum of trihydroxyethyl rutoside (99%) in this invention.

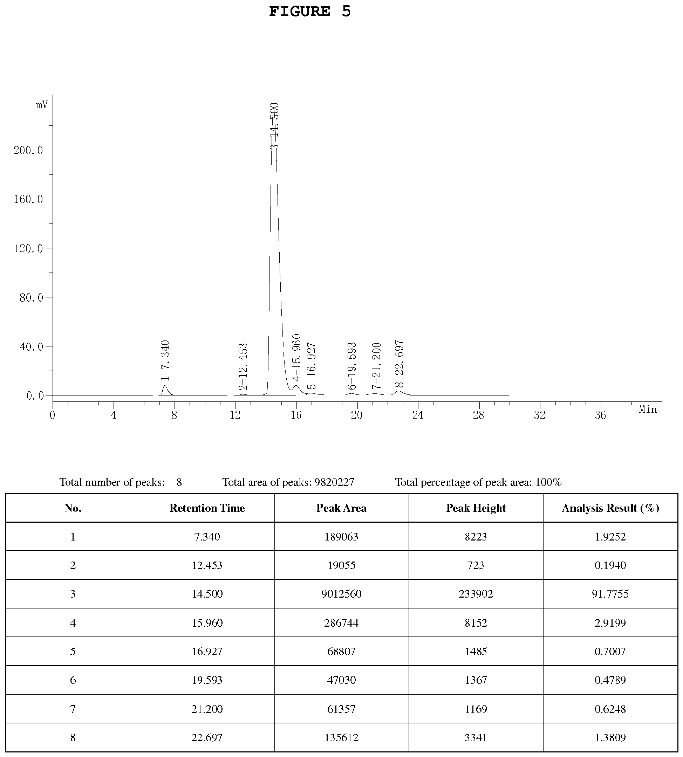

Figure 5: HPLC spectrum of trihydroxyethyl rutoside as final product in US Patent 6,855,697.

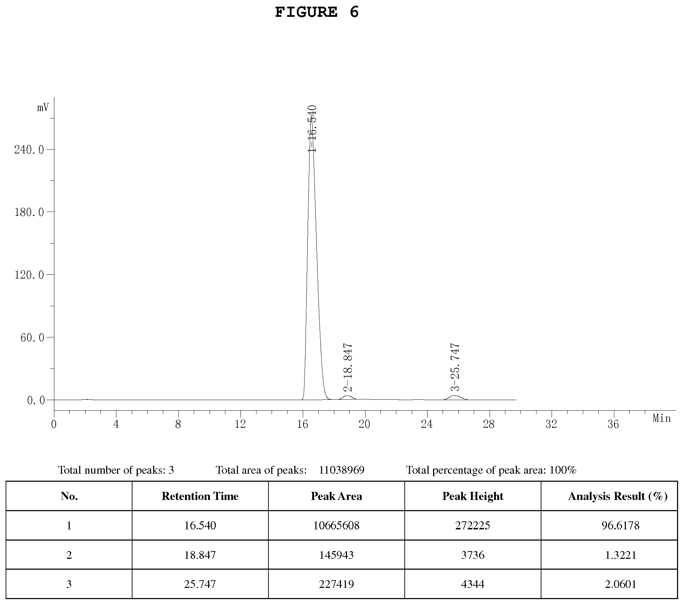

Figure 6: HPLC spectrum of trihydroxyethyl rutoside (96%) as reference substance on the market (Zhongjiansuo 100416-200503)

The following are illustrative examples only and are not meant to limit the scope of this invention.

### DETAILED DESCRIPTION OF THE INVENTION

In the present method, the preparation of 7-monohydroxyethyl rutoside in step (1) can utilize the technology in US patent 4,153,788, such as using borax to protect the hydroxyl groups at the 5, 3', and 4' positions. Alternatively, preparing 7-monohydroxyethyl rutoside from rutin can also employ the technology in US patent 3,430,815, i.e., synthesis in organic solvents.

During the preparation, hydroxyl protecting agent is used to protect the hydroxyl groups of rutin. Then, hydroxyethylation agent is added to the solvent to perform the reaction, wherein rutin can be purchased on the market, the hydroxyl protecting agent is borax, the hydroxyethylation agent is ethylene oxide, the reaction solvent is selected from water, methanol and ethanol, the reaction temperature is 30-50°C, the reaction time is 4-12 h, the reaction solution is let stand after reaction is complete, optionally at low temperature, the solution is made acidic if necessarily, and the resulting precipitate can be used for the next step after filtration.

The purification of 7-monohydroxyethyl rutoside in step (2) can use any industrially available method such as recrystallization, wherein 7-monohydroxyethyl rutoside is purified to a purity of greater than 95%, preferably greater than 98%, most preferably greater than 99%, and the purified product is used for further reaction.

The solvent for recrystallization is selected from water, methanol, ethanol, isopropanol and a mixture thereof. The recrystallization step includes dissolution, crystallization and filtration.

The preparation of trihydroxyethyl rutoside from purified 7-monohydroxyethyl rutoside in step (3) can utilize conventional hydroxyethylation approaches such as hydroxyethylation of 7-monohydroxyethyl rutoside by using ethylene oxide as hydroxyethylation agent. After reaction, the reaction product is passed through cation exchange resin and anion exchange resin to remove salts, or through macroporous resin to remove impurities. Then step (4) is performed.

In the process, a hydroxyethylation agent is added to react with 7-monohydroxyethyl rutoside, which is dissolved or dispersed in a solvent, wherein the hydroxyethylation agent is ethylene oxide, the reaction solvent is selected from water, methanol, ethanol, pyridine, and a mixture thereof, the reaction temperature is 50-80°C, the reaction time is 3-8 hours, the solution is let stand after reaction is complete, optionally at low temperature, the solution is made acidic if necessarily, and the resulting solution can be used for the next step.

Said cation exchange resins are selected from strong acidic polystyrene cationic exchange resins such as 007X7(732), 001X12, 001X1 and 001X16, said anion exchange resins are selected from strong basic polystyrene anion exchange resins such as 201X4(711) and 201X7(717). The method of passing the product through resins can be performed as follows: passing the solution through a column of resins, or adding the resins to the reaction solution, stirring the mixture, and then separating the crude product solution.

Said macroporous resins selected from D101, D201, DAB-8 and D301 can be used for purification in conventional manners, i.e., passing the filtered aqueous solution of the reaction product through a resin column, washing the column with purified water, and further washing with organic solvents of various concentrations in a gradient manner, wherein the organic solvent is ethanol, resulting in a product solution of crude trihydroxyethyl rutoside. The solution of crude product can be subjected to recrystallization after concentration and drying.

The purification step (4) utilizes recrystallization to prepare purified trihydroxyethyl rutoside, wherein the solvent for recrystallization is selected from water, methanol, ethanol, isopropanol and a mixture thereof, and the recrystallization step includes dissolution, crystallization and filtration.

Unless otherwise indicated, all the ratios and percentages are weight percentage values, and chromatographic purity refers to the results obtained according to the troxerutin analysis method in European Pharmacopoeia 7.0. Detailed preparation examples can be found in the examples below.

The advantages of this invention include the following.

Based on known reports, preparing highly pure trihydroxyethyl rutoside with purity greater than 98% is only possible using preparative chromatographic method, which is extremely expensive, strictly restrained within laboratory research, impossible for large-scale manufacture and wide application. The method disclosed in this invention takes a simpler method to obtain trihydroxyethyl rutoside with high purity, the cost is low and feasibility is great.

In comparison with current methods, this invention will greatly decrease cost. Especially for trihydroxyethyl rutoside, the cost is significantly decreased. The relevant experimental data are as follows.

The method in US Patent 3,420,815: according to this method, it is impossible to obtain trihydroxyethyl rutoside with a purity of 98%. The chromatographic purity of the product is 87.4% and the content is 84.2%. The color is dark yellow. Melting point is 178-182 °C. The cost is 400-500 Chinese Yuan per kilogram. With preparative chromatographic separation, trihydroxyethyl rutoside with a purity of 99% can be obtained. The cost, however, increases dramatically to 5,000-10,000 Chinese Yuan per gram.

In addition, the relevant data for comparison with the published preparation methods are as follows.

The method in US Patent 6,855,697: according to this method, it is impossible to obtain trihydroxyethyl rutoside with a purity of 98%. The chromatographic purity of the product is 92.1% and the content is 90.3%. The color is yellow. Melting point is 179-183 °C. The cost is 500-800 Chinese Yuan per kilogram. With preparative chromatographic separation, trihydroxyethyl rutoside with a purity of 99% can be obtained. The cost, however, increases dramatically to 5,000-10,000 Chinese Yuan per gram.

This invention provides a preparation of trihydroxyethyl rutoside with purity of 98%. Its color is light yellow, melting point is 185-187°C and the cost is around 2,000-3,000 Chinese Yuan per kilogram.

The invention provides a preparation of trihydroxyethyl rutoside with purity of 99%. Its color is light yellow, melting point is 186-188°C and the cost is around 3,000-5,000 Chinese Yuan per kilogram.

The products after step (1) and after purification in this invention are analyzed with results as below:

| Component | Before purification HPLC% | After purification HPLC % | Result comparison |
|---|---|---|---|
| 7-monohydroxyethyl rutoside | 91.35 | 98.72 | 7.37↑ |
| Impurity 1 R_{f}0.5 | 2.01 | Not detectable | 2.01↓ |
| Impurity 2 R_{f} 0.9 | 0.67 | Not detectable | 0.67↓ |
| Impurity 3 R_{f} 1.1 | 2.35 | 0.73 | 1.98↓ |
| Impurity 4 R_{f} 1.2 | 1.64 | 0.21 | 1.43↓ |
| Impurity 5 R_{f} 1.3 | 1.09 | 0.23 | 0.86↓ |
| Impurity 6 R_{f} 1.7 | 0.45 | 0.11 | 0.34↓ |
| Impurity 7 R_{f} 1.8 | 0.26 | Not detectable | 0.26↓ |

| | | | |
|---|---|---|---|
| Note: R_{f} is the retention time of impurity in reference to the main peak | | | |

According to the table above, it can be seen that the physicochemical property of 7-monohydroxyethyl rutoside is significantly different from the impurities 1-7 in the reaction. The effects of purification are significant

In order to prove the superiority of this invention, a comparison to the latest technology is also made.

Trihydroxyethyl rutoside is prepared by the method disclosed in US Patent 6,855,697. After reaction, the trihydroxyethyl rutoside in the reaction solution is purified by the following steps.

The solution containing trihydroxyethyl rutoside is passed through cation exchange resin 732, anion exchange resin 717 to remove salts, then concentrated under reduced pressure, and further spray-dried. With 8,000 ml of methanol added, the solid is heated to dissolve. After active charcoal for refinement of injection is added, the solution is heated at reflux for 30 min, and filtered while hot. The filtrate is let stand at room temperature for 6 h to allow crystallization. After filtration, the solid is dried at 40-50 °C and trihydroxyethyl rutoside is finally obtained, in which the content of trihydroxyethyl rutoside is tested to be 88.2%. The obtained solid was recrystallized from a 20-fold amount of methanol, and dried. The content of trihydroxyethyl rutoside is 90.6%.

The solution containing trihydroxyethyl rutoside is passed through cation exchange resin 732, anion exchange resin 711 to remove salts, then concentrated under reduced pressure, and further spray-dried. With 7,000 ml of methanol-water (37:3) added, the solid is heated to dissolve. After active charcoal for refinement of injection is added, the solution is heated at reflux for 30 min, and filtered while hot. The filtrate is let stand at room temperature for 6 h to allow crystallization. After filtration, the solid is dried at 40-50 °C and trihydroxyethyl rutoside is finally obtained, in which the content of trihydroxyethyl rutoside is tested to be 90.1%. The obtained solid was recrystallized from a 20-fold amount of methanol-water (37:3), and dried. The content of trihydroxyethyl rutoside is 91.6%.

The solution containing trihydroxyethyl rutoside is passed through cation exchange resin 001X14, anion exchange resin 717 to remove salts, then concentrated under reduced pressure, and further spray-dried. With 9000 ml of methanol-isopropanol (95:5) added, the solid is heated to dissolve. After active charcoal for refinement of injection is added, the solution is heated at reflux for 30 min, and filtered while hot. The filtrate is let stand at room temperature for 6 h to allow crystallization. After filtration, the solid is dried at 40-50 °C and trihydroxyethyl rutoside is finally obtained, in which the content of trihydroxyethyl rutoside is tested to be 91.7%. The obtained solid was recrystallized from a 20-fold amount of methanol-isopropanol (95:5), and dried. The content of trihydroxyethyl rutoside is 92.4%.

Trihydroxyethyl rutoside is prepared by the method disclosed in this invention. After reaction, the trihydroxyethyl rutoside in the reaction solution is purified by the following steps.

The solution containing trihydroxyethyl rutoside is passed through cation exchange resin 732, anion exchange resin 717 to remove salts, then concentrated under reduced pressure, and further spray-dried. With 8,000 ml methanol added, the solid is heated to dissolve. After active charcoal for refinement of injection is added and the solution is heated at reflux for 30 min, filtered while hot. The filtrate is then precipitated at room temperature for 6 h. After filtration, the solid is dried at 40-50 °C, and trihydroxyethyl rutoside is finally obtained, in which the content of trihydroxyethyl rutoside is tested to be 92.4%. The obtained solid was recrystallized from a 20-fold amount of methanol, and dried. The content of trihydroxyethyl rutoside is 98.2%.

The solution containing trihydroxyethyl rutoside is passed through cation exchange resin 732, anion exchange resin 711 to remove salts, then concentrated under reduced pressure, and further spray-dried. With 7,000 ml methanol-water (37:3) added, the solid is heated to dissolve. After active charcoal for refinement of injection is added and the solution is heated at reflux for 30 min, filtered while hot. The filtrate is then precipitated at room temperature for 6 h. After filtration, the solid is dried at 40-50 °C and trihydroxyethyl rutoside is finally obtained, in which the content of trihydroxyethyl rutoside is tested to be 93.5%. The obtained solid was recrystallized from a 20-fold amount of methanol-water (37:3), refined and dried. The content of trihydroxyethyl rutoside is 98.3%.

The solution containing trihydroxyethyl rutoside is passed through cation exchange resin 001X14, anion exchange resin 717 to remove salts, then concentrated under reduced pressure, and further spray-dried. After 9,000 ml methanol-isopropanol (95:5) added, the solid is heated to dissolve. After active charcoal for refinement of injection is added, the solution is heated at reflux for 30 min, and filtered while hot. The filtrate is then precipitated at room temperature for 6 h. After filtration, the solid is dried at 40-50 °C and trihydroxyethyl rutoside is finally obtained, in which the content of trihydroxyethyl rutoside is tested to be 95.8%. The obtained solid was recrystallized from a 20-fold amount of methanol-isopropanol (95:5), and dried. The content of trihydroxyethyl rutoside is 98.6%.

It can be seen from the comparison above that through the same purification method, the present preparation method gave much better result.

Based on existing technology such as the method in US Patent 6,855,697, the purity of trihydroxyethyl rutoside is 91.3%. Much purer product could not be obtained even by repeated recrystallization from a 20∼25-fold amount of methanol-isopropanol (95:5), as shown below:

| Number of Recrystallization | Initial | 1 | 3 | 5 | 8 |
|---|---|---|---|---|---|
| Purity | 91.77 | 92.43 | 93.51 | 94.36 | 95.21 |

### EXAMPLE 1

1. 328 g (0.86 mol) Borax (Na₂B₄O₇·10H₂O) is added into 2,500 ml deionized water, and dissolve with stirring. 605g (0.82 mol) rutin is added and dissolve with stirring at 40-45 °C. Clear and transparent solution of rutin-borax complex is obtained. Under 40-45 °C, with stirring, 88 g (2.0 mol) ethylene oxide is gradually introduced into the reaction solution followed by a reaction for about 6 h. The reaction is complete based on HPLC analysis. The pH value of the solution is adjusted to 2.0 by using 5N HCl and further let stand for 12 h at 3-5 °C. After filtration, the solid cake is obtained, in which contains 504 g 7-monohydroxyethyl rutoside and the yield is 95%.
2. 1,460g 7-monohydroxyethyl rutoside obtained from 1 above is added into 4,750 ml deionized water and heated to 60 °C with stirring. When full dissolution reached, the solution is filtrated and the filtrate stands at 3-5 °C for overnight. The predicated solid is obtained by filtration and further dried at 40-50 °C for 12 h. 450g 7-monohydroxyethyl rutoside is obtained. The content by anhydrous count is 98.3%. The chromatographic purity is 98.6%. The yield is 89.3% and the total yield is 84.8%.
3. 490 g (0.7mol) 7-monohydroxyethyl rutoside obtained from 2 above and 5.6 g sodium hydroxide are added into 1,880 ml deionized water, heated to and kept at 75-80 °C with stirring. 92 g (2.1 mol) ethylene oxide is gradually introduced into the reaction solution followed by a reaction reacting for 5-6 h. When the fraction of trihydroxyethyl rutoside reaches 75%-78% based on HPLC analysis, the supply of ethylene oxide is stopped. At the same time, nitrogen is introduced into the reaction and the temperature is rapidly lowered down. When the temperature is lower than 40 °C, the pH value is adjusted to 5.0±0.2 by using 3N HCl. The reaction solution is respectively passing through cation exchange resin column 732 and anion exchange resin column 717, followed by concentration under reduced pressure, and spray-dried. Solid powder of 520 g is obtained. The content of trihydroxyethyl rutoside is 92.3%. The melting point is 180-183 °C. The yield is 98% and the total yield is 83.1%.
4. 500 g solid powder as obtained from 3 above is added into 10,000 ml methanol. After an addition of 1.0 g active charcoal for refinement of injection, the solution is heated at reflux for 30 min, and filtered while hot. After a natural crystallization of filtrate at room temperature for 6 h, the solid is obtained by filtration and further dried at 40-50 °C. 410 g of trihydroxyethyl rutoside is obtained. The content is 96.8%. The yield is 82% and the total yield is 68.1%.
5. 410 g of the solid powder obtained from 4 above is added into 9,500 ml methanol. The solid is heated for dissolve. The solution is heated at reflux for 30 min and filtrated while hot. The filtrate gets through a natural precipitation of crystal at room temperature for 6 h. The solid is obtained after filtration and further dried at 40-50 °C under vacuum. 338 g of trihydroxyethyl rutoside is finally obtained. The content is 98.2%. The chromatographic purity is 98.4%. The melting point is 184-186 °C. The yield is 82.5% and the total yield is 56%.

### EXAMPLE 2

1. 164 g (0.43 mol) Borax (Na₂B₄O₇·10H₂O) and 12 g (0.3 mol) sodium hydroxide are added into 2,000 ml deionized water, and dissolve with stirring. With an addition of 605 g (0.82 mol) rutin, the solution is heated to and kept at 40-45 °C. On the condition of stirring, 88 g (2.0 mol) ethylene oxide is gradually introduced into the reaction solution followed by a reaction for about 12 h. The reaction is complete based on HPLC analysis. The pH value of the solution is adjusted to 2.0 by using 5N HCl and further let stand for 12h at 3-5 °C. After filtration, the solid cake is obtained, in which contains 510 g of 7-monohydroxyethyl rutoside and the yield is 96%.
2. 510 g 7-monohydroxyethyl rutoside obtained from 1 above (total weight is 1,450 g containing 940 g water) is added into 2,000 ml deionized water and heated to 40 °C under stirring. Saturated sodium bicarbonate solution is dropwise added until solid is fully dissolved. The solution is filtrated and the pH value of the filtrate is adjusted to 4.0 by using 0.1N HCl. The filtrate is let stand at 3-5 °C for overnight. The solid is obtained by filtration and further dried at 40-50 °C for 12h. 433 g 7-monohydroxyethyl rutoside (total weight 470 g) is obtained. The content by anhydrous count is 98.6%. The chromatographic purity is 98.8%. The yield is 85% and the total yield is 81.6%.
3. 433 g (0.67mol) 7-monohydroxyethyl rutoside obtained from 2 above and 5.6 g ammonium water are added into 1,880 ml deionized water, heated to and kept at 75-80 °C with stirring. 92 g (2.1 mol) ethylene oxide is gradually introduced into the reaction solution followed by a reaction for 5-6 h. When the fraction of trihydroxyethyl rutoside reaches 75-78% based on HPLC analysis, the supply of ethylene oxide is stopped. At the same time, nitrogen is introduced into the reaction and the temperature is rapidly lowered down. When the temperature is lower than 40 °C, the reaction solution is directly loaded onto the pre-treated macroporous resin D101 for purification, in which a total weight of 20 kg resin is used. When the loading is finished, deionized water is used for washing until the eluent is neutral and the chloride detection by silver nitrate is negative. Then 60% ethanol is used for washing until the eluent is colorless. Eluent is collected and concentrated to 2500 ml under reduced pressure. 465 g solid powder is obtained after spray drying. The content of trihydroxyethyl rutoside is 92%. The yield is 86% and the total yield is 70.2%
4. 465 g solid powder as obtained from 3 above is added into 8000 ml methanol and heated to dissolve. After an addition of 1.0 g active charcoal for refinement of injection and a refluxed for 30min, the solution is filtered while hot. After crystal precipitation of filtrate at room temperature for 6 h, the solid is obtained by filtration and further drying at 40-50 °C. The content is 97.2%. The yield is 82% and the total yield is 57.6%.
5. 381 g of the solid powder obtained from 4 above is added into 10500 ml methanol, and heated for dissolve. The solution is heated at reflux for 30 min and filtered while hot. The filtrate is let stand for a natural precipitation of crystal at room temperature for 6 h. The solid is obtained after filtration and further dried at 40-50 °C under vacuum. 314 g of trihydroxyethyl rutoside is finally obtained. The content is 98.3%. The chromatographic purity is 98.3%. The melting point is 184-186 °C. The yield is 82.5% and the total yield is 47.5%.

### EXAMPLE 3

1. 328 g (0.86 mol) Borax (Na₂B₄O₇·10H₂O) is added into 2,000 ml deionized water, and dissolve with stirring. 605g (0.82 mol) rutin is added and dissolved at 40-45 °C with stirring. Clear and transparent solution of rutin-borax complex is obtained. 88 g (2.0 mol) ethylene oxide is gradually introduced into reaction solution. After a reaction for about 12 h, the reaction is complete based on HPLC analysis. The reaction solution is directly loaded onto the pre-treated macroporous resin D101 for purification, in which a total weight of 25 kg resin is used. When the loading is finished, deionized water is used for washing until the eluent is neutral. The column is firstly washed by 10% ethanol, further washed by 10,000 ml 60% ethanol and finally washed by 90% ethanol. The eluent from 60% ethanol washing is collected and concentrated under reduced pressure until no smell of alcohol. The solution is diluted with water to a volume of 12,000 ml and stands at 3-5 °C for overnight. After filtration, the solid cake is further washed by icy water. 473 g trihydroxyethyl rutoside (total weight of 1,440 g) is obtained. The content by anhydrous count is 99.3%.The chromatographic purity is 99.3%. The yield is 89.3%.
2. 1,440 g solid containing water obtained from 1 above is added into 4,750 ml deionized water and heated to 60 °C under stirring. After full dissolution, the solution is filtered. Then, the solution is filtered and the pH value of the filtrate is adjusted to 2.0 by using 3N HCl. The filtrate is let stand at 3-5 °C for overnight. The solid is obtained by filtration and further dried at 40-50 °C for 12h. 463 g of 7-monohydroxyethyl rutoside (containing 8% water) is obtained. The content by anhydrous count is 99.5%. The chromatographic purity is 99.5%. The yield is 90.1% and the total yield is 80.5%.
3. 463 g (0.71mol) 7-monohydroxyethyl rutoside obtained from 2 above and 5.6 g sodium hydroxide are added into 1,880 ml deionized water, heated to and kept at 75-80 °C with stirring. 92 g (2.1 mol) ethylene oxide is gradually introduced into the reaction solution followed by a reaction for 5-6 h. When the fraction of trihydroxyethyl rutoside based on HPLC analysis reaches 75%-78%, the supply of ethylene oxide is stopped. At the same time, nitrogen is introduced into the reaction and the temperature is rapidly lowered down. When the temperature is lower than 40 °C, the pH value is adjusted to 5.0±0.2 by using 3N HCl. The reaction solution is respectively passed through cation exchange resin column 732 and anion exchange resin column 717 to remove salts, followed by concentration under reduced pressure, and spray-dried. Solid powder of 531 g is obtained. The content of trihydroxyethyl rutoside is 85%. The melting point is 180-183 °C. The yield is 85% and the total yield is 68.4%.
4. 465 g solid powder as obtained from 3 above is added into 8,000 ml methanol and heated to dissolve. After an addition of 1.0 g active charcoal for refinement of injection, the solution is heated at reflux for 30min, and filtered while hot. After a natural precipitation of crystal precipitation at room temperature for 6 h, the solid is obtained by filtration and further drying at 40-50 °C. 531 g of trihydroxyethyl rutoside is obtained. The content is 95.8%. The yield is 82% and the total yield is 56%.
5. 435 g of the solid powder obtained from 4 above is added into 10,500 ml methanol, and heated to dissolve. The solution is heated at reflux for 30 min, and filtrated while hot. After a natural crystal precipitation at room temperature for 6h, the solid is obtained after filtration and further dried at 40-50 °C under vacuum. Finally, 354 g trihydroxyethyl rutoside is obtained. The content is 98.4%. The chromatographic purity is 98.4%. The melting point is 184-186 °C. The yield is 81.5% and the total yield is 45.6%.

### EXAMPLE 4

1. The preparation of 7-monohydroxyethyl rutoside is the same as descried in EXAMPLE 1.
2. 510 g (0.71 mol) 7-monohydroxyethyl rutoside obtained from 1 above and 5.6 g sodium hydroxide are added into the mixture solution of 1,000 ml deionized water and 800 ml ethanol. The solution is heated to and kept at 70-75 °C under stirring. 92 g (2.1 mol) ethylene oxide is gradually introduced into reaction solution followed by a reaction for 10-13 h. When the fraction of trihydroxyethyl rutoside reaches 75-78% based on HPLC analysis, the supply of ethylene oxide is stopped. At the same time, nitrogen is introduced into the reaction and the temperature is rapidly lowered down. When the temperature is lower than 40 °C, the pH value is adjusted to 5.0±0.2 by using 3N HCl. The ethanol is recycled by vacuum distillation. The reaction solution is respectively passed through cation exchange resin column 732 and anion exchange resin column 717 to remove salts, followed by spray drying. Solid powder of 480 g is obtained. The content of trihydroxyethyl rutoside is 93.4%. The melting point is 181-184 °C. The yield is 85%.
3. The obtained solid from 2 above was recrystallized according to the method in EXAMPLE 1 by using methanol. 323 g of trihydroxyethyl rutoside is obtained. The chromatographic purity is 98.6%. The melting point is 185-186 °C. The total yield is 53.5%.

### EXAMPLE 5

1. The preparation of 7-monohydroxyethyl rutoside is the same as descried in EXAMPLE 1.
2. 510 g (0.71 mol) 7-monohydroxyethyl rutoside and 5.6 g sodium hydroxide are added into the mixture solution of 1,000 ml deionized water and 800 ml methanol. The solution heated to and kept at 60-70 °C under stirring. 92 g (2.1 mol) ethylene oxide is gradually introduced into reaction solution followed by a reaction for 10-13 h. When the fraction of trihydroxyethyl rutoside reaches 75-78% based on HPLC analysis, the supply of ethylene oxide is stopped. At the same time, nitrogen is introduced into the reaction and the temperature is rapidly lowered down. When the temperature is lower than 40 °C, the pH value is adjusted to 5.0±0.2 by using 3N HCl. The reaction solution is respectively passed through cation exchange resin column 732 and anion exchange resin column 717 to remove salts. The methanol is recycled by vacuum distillation. 474 g of solid powder of is obtained after spray drying. The content of trihydroxyethyl rutoside is 94.5%. The melting point is 181-184 °C. The yield is 84%.
3. The solid as obtained from 2 above was recrystallized according to the method 1 in EXAMPLE 2 by using methanol. 317 g of trihydroxyethyl rutoside is obtained. The chromatographic purity is 98.7%. The melting point is 185-186 °C. The total yield is 52.5%

### EXAMPLE 6

1. The preparation of 7-monohydroxyethyl rutoside is the same as descried in EXAMPLE 1.
2. Within a 5,000 ml autoclave, 510 g (0.71 mol) 7-monohydroxyethyl rutoside, 10 ml trimethylamine, 2,000 ml methanol, and 132 g (3.0 mol) ethylene oxide at last are added. Once the addition is finished, the autoclave is sealed immediately and heated to and kept at 75-80 °C under stirring followed by a reaction for 2-3h. When the temperature is lower than 40 °C and the pressure is released, the reaction solution is filtrated. The filtrated solution is respectively passed through the cation exchange resin 732 column and the anion exchange resin 717 column. The pH value of the solution is adjusted to 5.0±0.2 by using 5N HCl. The filtrate is let stand for 6-8 h at room temperature. Crystal is obtained after filtration and the solid cake of 380 g is obtained.
3. The solid cake obtained from 2 above is re-dissolved in a 20-fold amount of hot methanol. The solution is heated at flux for 30 min and filtered. The filtrate is let stand for 6-8 h at room temperature. After filtration, the crystal is dried under vacuum at 40-50 °C. The content of trihydroxyethyl rutoside is 98.1%. The melting point is 185-186 °C. The yield is 58%.

### EXAMPLE 7

1. The preparation of 7-monohydroxyethyl rutoside is the same as descried in EXAMPLE 1.
2. Within a 5,000 ml autoclave, 510 g (0.71 mol) 7-monohydroxyethyl rutoside, 10 ml diethylamine, 2,000 ml methanol, and 132 g (3.0 mol) ethylene oxide as the last are added. Once the addition is finished, the autoclave is sealed immediately and heated to and kept at 75-80 °C under stirring followed by a reaction for 2-3h. When the temperature is lower than 40 °C and the pressure is released, the reaction solution is filtered. The filtrate is respectively passed through the cation exchange resin 732 column and the anion exchange resin 717 column. The pH value of the solution is adjusted to 5.0±0.2 by using 5N HCl. The filtrate is let stand for 6-8 h at room temperature. Crystal is obtained after filtration and a solid cake of 375 g is obtained.
3. The solid cake obtained from 2 above is re-dissolved in a 20-fold amount of hot methanol-isopropanol (37:3). The solution is heated at reflux for 30 min, and filtered. The filtrate is let stand for 6-8 h at room temperature. Crystal is obtained after filtration followed by vacuum drying at 40-50 °C. A solid powder of 302 g is obtained. The content of trihydroxyethyl rutoside is 98.3%. The melting point is 184-186 °C. The yield is 57%.

### EXAMPLE 8

1. The preparation of 7-monohydroxyethyl rutoside is the same as descried in EXAMPLE 1.
2. Within a 5,000 ml autoclave, 510 g (0.71 mol) 7-monohydroxyethyl rutoside, 30 ml ammonium water, 2,000 ml methanol, and 154 g (3.5 mol) ethylene oxide are added. Once the addition is finished, the autoclave is sealed immediately and heated to and kept at 75-80 °C with stirring followed by a reaction for 2-3h. When the temperature is lower than 40 °C and the pressure is released, the reaction solution can be filtered. The filtrated solution respectively passes through the cation exchange resin 732 column and the anion exchange resin 717 column. The pH value of the solution is adjusted to 5.0±0.2 by using 5N HCl. The filtrate is let stand for 6-8 h at room temperature. Crystal is obtained after filtration and 360 g of solid cake is obtained.
3. The solid cake obtained from 2 above is re-dissolved in a 20-fold amount of hot methanol. The solution is heated at reflux for 30 min and is filtrated. The filtrate is let stand for 6-8 h at room temperature. Crystal is obtained after filtration followed by vacuum drying at 40-50 °C. 290 g of solid powder is obtained. The content of trihydroxyethyl rutoside is 98.2%. The melting point is 184-186 °C. The yield is 55%.

### EXAMPLE 9

1. The preparation of 7-monohydroxyethyl rutoside is the same as descried in EXAMPLE 1.
2. Within a 5,000 ml autoclave, 510 g (0.71 mol) 7-monohydroxyethyl rutoside, 10 ml pyridine, 1,600 ml methanol, 400 ml methanol and 154 g (3.5 mol) ethylene oxide as the last are added. Once the addition is finished, the autoclave is sealed immediately and heated to and kept at 75-80 °C under stirring followed by a reaction for 2-3h. When the temperature is lower than 40 °C and the pressure is released, the reaction solution is filtered. The filtrate is respectively passed through the cation exchange resin 732 column and the anion exchange resin 717 column. The pH value of the solution is adjusted to 5.0±0.2 by using 5N HCl. The filtrate is let stand for 6-8 h at room temperature. Crystal is obtained after filtration and 420 g of solid cake is obtained.
3. The solid cake obtained from 2 above is re-dissolved in a 20-fold amount of methanol-ethanol (95:5) and heated to dissolve. The solution is heated at reflux for 30 min and filtrated. The filtrate is let stand for 6-8 h at room temperature. Crystal is obtained after filtration followed by a vacuum drying at 40-50 °C. The 336 g of solid powder is obtained. The content of trihydroxyethyl rutoside is 98.2%. The melting point is 184-186 °C. The yield is 64%.

### EXAMPLE 10

1. The preparation of 7-monohydroxyethyl rutoside is the same as descried in EXAMPLE 1.
2. Within a 5,000 ml autoclave, 510 g (0.71 mol) 7-monohydroxyethyl rutoside, 10 ml pyridine, 2,000 ml methanol, and 154 g (3.5 mol) ethylene oxide as the last are added. Once the addition is finished, the autoclave is sealed immediately and heated to and kept at 75-80 °C with stirring followed by a reaction for 2-3h. When the temperature is lower than 40 °C and the pressure is released, the pH value of the solution is adjusted to 5.0±0.2 by using 5N HCl. The filtrate stands for 6-8 h at room temperature. Crystal is obtained after filtration and 410 g of solid cake is obtained.
3. The solid cake obtained from 2 above is re-dissolved in a 20-fold amount of methanol and heated to dissolve. The solution is heated at reflux for 30 min and filtrated. The filtrate is let stand for 6-8 h at room temperature. Crystal is obtained after filtration followed by vacuum drying at 40-50 °C. A solid powder of 310 g is obtained. The content of trihydroxyethyl rutoside is 98.3%. The melting point is 184-186 °C. The yield is 61%.

### EXAMPLE 11

1. 100 g trihydroxyethyl rutoside with a purity of 98.7% as obtained from EXAMPLE 5 is added into 3,000 ml methanol-ethanol. The solution is heated at reflux for 30 min, and filtrated. After filtration, the crystal is dried under vacuum at 40-50 °C. 80.2 g of trihydroxyethyl rutoside is obtained. The chromatographic purity is 98.8%. The content is 98.8%. The melting point is 186-188 °C.
2. 80 g trihydroxyethyl rutoside with a purity of 98.8% as obtained above is added into 2,400 ml methanol-isopropanol (95:5). The solution is heated at reflux for 30 min, and filtrated while hot. The filtrate is let stand for 6-8 h at room temperature. After filtration, the crystal is dried under vacuum g at 40-50 °C. 64.3 g trihydroxyethyl rutoside is obtained. The chromatographic purity is 99.2%. The content is 99.2%. The melting point is 187-189 °C.

## Claims

1. A preparation method of trihydroxyethyl rutoside, **characterized by** that 7-monohydroxyethyl rutoside is first prepared by ethylation of rutin, purified thereafter, and then converted to trihydroxyethyl rutoside by hydroxyethylation, wherein:
in the step of preparation of 7-monohydroxyethyl rutoside, the hydroxyl groups of rutin are protected by a hydroxyl-protecting agent before reaction with a hydroxyethylation agent in a solvent, wherein the hydroxyl-protecting agent is borax, the hydroxyethylation agent is ethylene oxide, the solvent is selected from water, methanol and ethanol, the reaction temperature is 30-50°C and the reaction time is 4-14 hours;
in the step of purification of 7-monohydroxyethyl rutoside, a recrystallization method is used for purification, wherein the solvent for recrystallization is selected from water, methanol, ethanol, isopropanol, and a mixture thereof, and wherein the recrystallization method includes dissolution, crystallization and filtration;
in the step of preparation of trihydroxyethyl rutoside from purified 7-monohydroxyethyl rutoside, a hydroxyethylation agent is added after dissolving or dispersing 7-monohydroxyethyl rutoside in a solvent, wherein the hydroxyethylation agent is ethylene oxide, the solvent is selected from water, methanol, ethanol, pyridine and a mixture thereof, a catalyst is selected from sodium hydroxide, potassium hydroxide and ammonium water, the reaction temperature is 50-80°C and the reaction time is 3-8 hours, and the product is passed through cation and anion resins, or is treated with macroporous resin after the reaction is complete; wherein said cationic resins are selected from strong acidic polystyrene cation exchange resins, such as 007X7(732), 001X12, 001X1 and 001X16, the anion resins are selected from strong basic polystyrene anion exchange resins, such as 201X4(711) and 201X7(717); wherein said macroporous resins are selected from non-polar polystyrene macroporous resins, such as D101, Shanshi101, and low polar macroporous resins, such as D_{A}, DAB-8 and D301;
in the step of purification of trihydroxyethyl rutoside, a recrystallization method is used for purification, wherein a solvent for recrystallization is selected from water, methanol, ethanol, isopropanol, and a mixture thereof, and wherein the recrystallization method includes dissolution, crystallization and filtration.

2. The preparation method of Claim 1, comprising the following steps:
(1) Preparation of 7-monohydroxyethyl rutoside from rutin;
(2) Purification of 7-monohydroxyethyl rutoside;
(3) Preparation of trihydroxyethyl rutoside from purified 7-monohydroxyethyl rutoside;
(4) Purification of trihydroxyethyl rutoside.

3. The preparation method of Claim 1, wherein 7-monohydroxyethyl rutoside is purified to a purity of at least 95 weight percent, before further reaction.

4. The method of Claim 3, wherein 7-monohydroxyethyl rutoside is purified to a purity of at least 98 weight percent, before further reaction.

5. The method of Claim 4, wherein 7-monohydroxyethyl rutoside is purified to a purity of at least 99 weight percent, before further reaction.

6. The preparation method of Claim 5, wherein:
in the step of preparation of 7-monohydroxyethyl rutoside, the hydroxyl-protecting agent is borax, the hydroxyethylation agent is ethylene oxide, the solvent is selected from water, methanol and ethanol, the reaction temperature is 30-50°C and the reaction time is 4-12 hours;
in the step of purification of 7-monohydroxyethyl rutoside, a recrystallization method is used for purification, wherein the solvent for recrystallization is selected from water, methanol, ethanol, and a mixture thereof;
in the step of preparation of trihydroxyethyl rutoside from purified 7-monohydroxyethyl rutoside, the hydroxyethylation agent is ethylene oxide, the solvent is selected from water, methanol, ethanol, and a mixture thereof, the reaction temperature is 50-80°C and the reaction time is 3-13 hours, and the product is passed through cation and anion resins to remove salts, or treated with macroporous resin to remove impurities after the reaction is complete;
in the step of purification of trihydroxyethyl rutoside, recrystallization is used, wherein the recrystallization solvent is selected from water, methanol, ethanol, and a mixture thereof.

7. The preparation method of Claim 1, wherein the hydroxyethylated product contains less than 2 weight percent of non-hydroxyethylated rutoside derivatives.

8. The preparation method of Claim 1, wherein the final trihydroxyethyl rutoside contains less than 2 weight percent of impurities.

9. The preparation method of Claim 8, wherein the final trihydroxyethyl rutoside contains less than 1 weight percent of impurities.

## Patentansprüche

1. Zubereitungsverfahren für Trihydroxyethylen-Rutosid, **dadurch gekennzeichnet, dass** 7-Monohydroxyethylen-Rutosid zuerst durch Ethylierung von Rutin zubereitet wird, dann gereinigt wird, und dann durch Hydroxyethylierung in Trihydroxyethylen-Rutosid umgewandelt wird, wobei:
Im Schritt der Zubereitung von 7- Monohydroxyethylen-Rutosid die Hydroxyl-Gruppen von Rutin vor der Reaktion mit einer Hydroxyethylierungsmittel in einer Lösung durch ein Hydroxyl-Schutz-Mittel geschützt werden, wobei das Hydroxyl-Schutz-Mittel Borax ist, das Hydroxyethylierungsmittel Ethylenoxid ist, das Lösungsmittel wird ausgewählt aus Wasser, Methanol und Ethanol, die Reaktionstemperatur ist 30-50°C und die Reaktionszeit ist 4-14 Stunden;
Im Schritt des Reinigens von 7-Monohydroxyethylen-Rutosid wird für die Reinigung ein Rekristallisierungsverfahren verwendet, wobei das Lösungsmittel für die Rekristallisierung ausgewählt wird aus Wasser, Methanol, Ethanol, Isopropanol, und einer Mischung davon, und wobei das Rekristallisierungsverfahren Auflösen, Kristallisation und Filtration umfasst;
Im Schritt der Zubereitung von Trihydroxyethylen-Rutosid aus gereinigtem 7-Monohydroxyethylen-Rutosid nach dem Auflösen oder Dispergieren von 7-Monohydroxyethylen-Rutosid in einem Lösungsmittel ein Hydroxyethylierungsmittel hinzugefügt wird, wobei das Hydroxyethylierungsmittel Ethylenoxid ist, das Lösungsmittel ausgewählt wird aus Wasser, Methanol, Ethanol, Pyridin und einer Mischung davon, ein Katalysator ausgewählt wird aus Natriumhydroxid, Kaliumhydroxid und Ammoniumwasser, die Reaktionstemperatur 50-80°C beträgt und die Reaktionszeit 3-8 Stunden beträgt, und das Produkt durch Kationen- und Anionen-Kunstharze hindurchgeht oder, nachdem die Reaktion vollständig ist, mit makroporösem Kunstharz behandelt wird; wobei die kationischen Harze ausgewählt sind aus stark sauren Polystyren-Kationen-Austauschharzen, so wie 007X7(732), 001X12, 001X1 und 001X16, die Anionen-Harze ausgewählt werden aus stark basischen Polystyren-Anionen-Austauschharzen, so wie 201X4 (711) und 201X7 (717); wobei die makroporösen Harze ausgewählt werden aus nicht-polaren Polystyrol makroporösen Harzen, so wie D101, Shanshi 101, und niedrig polaren makroporösen Harzen, so wie D_{A}, DAB-8 und D301;
im Schritt des Reinigens von Trihydroxyethylen-Rutosid ein Rekristallisierungsverfahren für die Reinigung eingesetzt wird, wobei ein Lösungsmittel für die Rekristallisierung ausgewählt wird aus Wasser, Methanol, Ethanol, Isopropanol, und einer Mischung davon, und wobei das Rekristallisierungsverfahren Auflösen, Kristallisieren und Filtrieren einschließt.

2. Zubereitungsverfahren nach Anspruch 1, umfassend die folgende Schritte:
(1) Zubereiteten von 7- Monohydroxyethylen-Rutosid aus Rutin;
(2) Reinigen von 7- Monohydroxyethylen-Rutosid;
(3) Zubereiten von Trihydroxyethylen-Rutosid aus gereinigtem 7- Monohydroxyethylen-Rutosid;
(4) Reinigen von Trihydroxyethylen-Rutosid.

3. Zubereitungsverfahren nach Anspruch 1, wobei 7-Monohydroxyethylen-Rutosid bis zu einer Reinheit von wenigstens 95 Gewichts-% gereinigt wird, vor einer weiteren Reaktion.

4. Zubereitungsverfahren nach Anspruch 3, wobei 7-Monohydroxyethylen-Rutosid bis zu eine Reinheit von wenigstens 98 Gewichts-Prozent gereinigt wird, vor einer weiteren Reaktion.

5. Zubereitungsverfahren nach Anspruch 4, wobei 7-Monohydroxyethylen-Rutosid bis zu einer Reinheit von wenigstens 99 Gewichts-% gereinigt wird, vor einer weiteren Reaktion.

6. Zubereitungsverfahren nach Anspruch 5, wobei:
Im Schritt der Zubereitung von 7-Monohydroxyethylen-Rutosid das Hydroxyl-Schutz-Mittel Borax ist, das Hydroxyethylationsmittel Ethylenoxid ist, das Lösungsmittel ist ausgewählt aus Wasser, Methanol und Ethanol, die Reaktionstemperatur beträgt 30-50°C und die Reaktionszeit ist 4-12 Stunden;
Im Schritt der Reinigung von 7-Monohydroxyethylen-Rutosid ein Rekristallisierungsverfahren für die Reinigung verwendet wird, wobei das Reinigungsmittel für die Rekristallisierung ausgewählt wird aus Wasser, Methanol, Ethanol, und einer Mischung davon;
Im Schritt der Zubereitung von Trihydroxyethylen-Rutosid aus gereinigtem 7-Monohydroxyethylen-Rutosid das Hydroxyethylationsmittel Ethylenoxid ist, das Lösungsmittel ausgewählt wird aus Wasser, Methanol, Ethanol und einer Mischung davon, die Reaktionstemperatur 50-80°C beträgt und die Reaktionszeit 3 bis 13 Stunden ist, und das Produkt durch kationische und anionische Harze hindurchgeht, um Salze zu entfernen, oder mit Makroporösen Harz behandelt wird, um Salze zu entfernen, oder, nachdem die Reaktion vollständig ist, mit makroporösen Harzen behandelt wird, um Verunreinigungen zu entfernen;
Im Schritt der Reinigung von Trihydroxyethylen-Rutosid Rekristallisierung eingesetzt wird, wobei das Rekristallisierungs-Lösungsmittel ausgewählt wird aus Wasser, Methanol, Ethanol, und einer Mischung davon.

7. Zubereitungsverfahren nach Anspruch 1, wobei das hydroxyethylierte Produkt weniger als 2 Gewichts-% nicht hydroxyethylierter Rutosid-Derivate enthält.

8. Zubereitungsverfahren nach Anspruch 1, wobei das finale Trihydroxyethylen-Rutosid weniger als 2 Gewichts-% Verunreinigungen enthält.

9. Zubereitungsverfahren nach Anspruch 8, wobei das Trihydroxyethylen-Rutosid weniger als 1 Gewichts-% Verunreinigungen enthält.

## Revendications

1. Procédé de préparation de trihydroxyéthyl-rutoside, **caractérisé en ce que** le 7-monohydroxyéthil-rutoside est tout d'abord préparé par éthylation de rutine, puis est purifié et est ensuite converti en trihydroxyéthyl-rutoside par hydroxyéthylation, dans lequel
dans l'étape de préparation du trihydroxyéthyl-rutoside les groupes hydroxyles de rutine sont protégés par un agent protecteur de groupe hydroxyle avant la réaction avec un agent d'hydroxyéthylation dans un solvant, l'agent protecteur de groupe hydroxyle est le borax, l'agent d'hydroxyéthylation est l'oxyde d'éthylène, le solvant est sélectionné parmi l'eau, le méthanol et l'éthanol, la température de réaction est de 30 à 50 °C et le temps de réaction est de 4 à 14 heures ;
dans l'étape de purification du 7-monohydroxyéthil-rutoside, un procédé de recristallisation est utilisé pour la purification, le solvant pour la recristallisation est sélectionné parmi l'eau, le méthanol, l'éthanol, l'isopropanol et un mélange de ceux-ci et le procédé de recristallisation comprend la dissolution, la cristallisation et la filtration ;
dans l'étape de préparation du trihydroxyéthyl-rutoside à partir de 7-monohydroxyéthil-rutoside purifié, un agent d'hydroxyéthylation est ajouté après dissolution ou dispersion du 7-monohydroxyéthil-rutoside dans un solvant, l'agent d'hydroxyéthylation est l'oxyde d'éthylène, le solvant est sélectionné parmi l'eau, le méthanol, l'éthanol, la pyridine et un mélange de ceux-ci, un catalyseur est sélectionné parmi l'hydroxyde de sodium, l'hydroxyde de potassium et l'eau d'ammonium, la température de réaction est de 50 à 80 °C et le temps de réaction est de 3 à 8 heures et on fait passer le produit par des résines de cations et d'anions ou on le traite avec une résine macroporeuse après que la réaction soit terminée, lesdites résines cationiques étant sélectionnées parmi les résines de polystyrène échangeuses de cations fortement acides telles que 007X7(732), 001 X12, 001 X1 et 001X16, les résines d'anions étant sélectionnées parmi les résines de polystyrène échangeuses d'anions fortement acides telles que 201X4(711) et 201 X7(717), lesdites résines macroporeuses étant sélectionnées parmi les résines macroporeuses de polystyrène non polaires telles que D101, Shanshi101 et les résines macroporeuses faiblement polaires telles que D_{A}, DAB-8 et D301 ;
dans l'étape de purification du trihydroxyéthyl-rutoside, un procédé de recristallisation est utilisé pour la purification, un solvant pour la recristallisation est sélectionné parmi l'eau, le méthanol, l'éthanol, l'isopropanol et un mélange de ceux-ci et le procédé de recristallisation comprend la dissolution, la cristallisation et la filtration.

2. Procédé de préparation selon la revendication 1 comprenant les étapes suivantes :
(1) Préparation de 7-monohydroxyéthil-rutoside à partir de rutine ;
(2) Purification de 7-monohydroxyéthil-rutoside ;
(3) Préparation de trihydroxyéthyl-rutoside à partir de 7-monohydroxyéthil-rutoside purifié ;
(4) Purification de trihydroxyéthyl-rutoside.

3. Procédé de préparation selon la revendication 1, le7-monohydroxyéthil-rutoside étant purifié à une pureté d'au moins 95 % en poids avant une autre réaction.

4. Procédé selon la revendication 3, le 7-monohydroxyéthil-rutoside étant purifié à une pureté d'au moins 98 % en poids avant une autre réaction.

5. Procédé selon la revendication 4, le 7-monohydroxyéthil-rutoside étant purifié à une pureté d'au moins 99 % en poids avant une autre réaction.

6. Procédé de préparation selon la revendication 5 dans lequel :
dans l'étape de préparation du 7-monohydroxyéthil-rutoside, l'agent protecteur de groupe hydroxyle est le borax, l'agent d'hydroxyéthylation est l'oxyde d'éthylène, le solvant est sélectionné parmi l'eau, le méthanol et l'éthanol, la température de réaction est de 30 à 50 °C et le temps de réaction est de 4 à 12 heures ;
dans l'étape de purification du 7-monohydroxyéthil-rutoside, un procédé de recristallisation est utilisé pour la purification, le solvant pour la recristallisation est sélectionné parmi l'eau, le méthanol, l'éthanol et un mélange de ceux-ci ;
dans l'étape de préparation du trihydroxyéthyl-rutoside à partir de 7-monohydroxyéthil-rutoside purifié, l'agent d'hydroxyéthylation est l'oxyde d'éthylène, le solvant est sélectionné parmi l'eau, le méthanol et l'éthanol et un mélange de ceux-ci, la température de réaction est de 50 à 80 °C et le temps de réaction est de 3 à 13 heures et on fait passer le produit par des résines de cations et d'anions pour enlever les sels ou on le traite avec de la résine macroporeuse pour enlever les impuretés après que la réaction soit terminée ;
dans l'étape de purification du trihydroxyéthyl-rutoside, un procédé de recristallisation est utilisé, le solvant pour la recristallisation est sélectionné parmi l'eau, le méthanol, l'éthanol et un mélange de ceux-ci.

7. Procédé de préparation selon la revendication 1, le produit hydroxyéthylé contenant moins de 2 % en poids de dérivés de rutoside non hydroxyéthylés.

8. Procédé de préparation selon la revendication 1, le trihydroxyéthyl-rutoside final contenant moins de 2 % en poids d'impuretés.

9. Procédé de préparation selon la revendication 8, le trihydroxyéthyl-rutoside final contenant moins d'1 % en poids d'impuretés.
